(19) **Europäisches Patentamt / European Patent Office / Office européen des brevets**

(11) **EP 3 955 707 A1**

(12) **EUROPEAN PATENT APPLICATION**

(43) Date of publication:
**16.02.2022 Bulletin 2022/07**

(21) Application number: **20190781.3**

(22) Date of filing: **12.08.2020**

(51) International Patent Classification (IPC):
*H05G 1/54* (2006.01)  *G16H 40/40* (2018.01)
*A61B 6/00* (2006.01)

(52) Cooperative Patent Classification (CPC):
**H05G 1/54; G16H 40/40;** A61B 6/586

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Danlex EOOD**
**1619 Sofia (BG)**

(72) Inventor: **ZOGRAFOV, Nikolay**
**1582 Sofia (BG)**

(74) Representative: **KLIMENT & HENHAPEL**
**Patentanwälte OG**
**Gonzagagasse 15/2**
**1010 Wien (AT)**

(54) **METHOD AND SYSTEM FOR PREDICTING A FAILURE PROBABILITY OF A COMPONENT OF AN X-RAY SYSTEM**

(57) The invention relates to a method for predicting a failure probability of a component of an X-ray system, comprising the following steps:

i. gathering data from maintenance reports and/or maintenance archives relating to the X-ray system and/or to its component, and from sensors arranged inside the X-ray system as well as from sensors arranged outside of the X-ray system;

ii. statistically processing said data in order to obtain at least one threshold value for at least one operating parameter of the component, and/or providing a predefined threshold value for at least one further operating parameter of the component;

iii. dynamically adapting the at least one threshold value based, at least, on a difference between and/or a ratio of sensor measurements from the inside of the X-ray system and from the outside of the X-ray system;

iv. monitoring the at least one operating parameter of the component and/or the at least one further operating parameter of the component;

v. comparing the values of the monitored at least one operating parameter and/or the monitored at least one further operating parameter to the corresponding threshold value;

vi. based on said comparison, determining the failure probability of the component.

FIG. 1

**Description**

FIELD OF THE INVENTION

The present invention relates to a method for predicting a failure probability of a component of an X-ray system.

Furthermore, the invention relates to a system being adapted to execute said method.

STATE OF THE ART

Methods for monitoring the operation of an X-ray system are generally known. Whereas most of these are designed to merely detect an existing malfunction of the X-ray system and to subsequently, directly or indirectly, trigger repair or service works on said X-ray system, some methods try to implement the concept of predictive maintenance. By employing such methods, the probability of future technical failures of certain components (e.g. X-ray generators) of X-ray systems, which systems are commonly used for inspection of luggage, containers, cargo, mail, vehicles, trucks, train sets and the like, can be determined, allowing for spare parts to be obtained and installed just before the actual malfunction occurs. In this way, downtimes of X-ray systems as well as storage capacities required for storing spare parts can be significantly reduced.

US 6,453,009 B2, for example, discloses a method and a system for predicting failure of an X-ray tube, where a plurality of operating parameters of the X-ray tube are monitored, and a failure prediction value is derived from the monitored parameters. The failure prediction value is then compared to a reference value. Based upon this comparison, a signal indicative of predicted tube failure is generated. The failure prediction value may be derived from the monitored parameters by means of discriminant analysis.

Moreover, according to US 6,453,009 B2, the parameters may be monitored, the comparison made, and the failure prediction signal generated at a remote location. While most of the operating parameters are physical quantities directly measured by internal sensors of the X-ray system, also parameters derived from spit events/spit rate exceeded errors in the X-ray system are taken into account.

OBJECTIVE OF THE INVENTION

The present invention aims to provide an improved method for predictive maintenance of X-ray systems, facilitating determination of future failure of critical system components with higher accuracy and reliability.

Moreover, a system adapted to perform said method shall be provided, allowing for predictive maintenance to be performed on X-ray systems which, themselves, have not been equipped with the means necessary for such methods to be employed.

PRESENTATION OF THE INVENTION

An objective of the present invention is achieved by a method for predicting a failure probability of a component of an X-ray system, comprising the following steps:

> i. gathering data from maintenance reports and/or maintenance archives relating to the X-ray system and/or to its component, and from sensors arranged inside the X-ray system as well as from sensors arranged outside of the X-ray system;

> ii. statistically processing said data in order to obtain at least one threshold value for at least one operating parameter of the component, and/or providing a predefined threshold value for at least one further operating parameter of the component;

> iii. dynamically adapting the at least one threshold value based, at least, on a difference between and/or a ratio of sensor measurements from the inside of the X-ray system and from the outside of the X-ray system;

> iv. monitoring the at least one operating parameter of the component and/or the at least one further operating parameter of the component;

> v. comparing the values of the monitored at least one operating parameter and/or the monitored at least one further operating parameter to the corresponding threshold value;

> vi. based on said comparison, determining the failure probability of the component.

This method for early warning and predictive maintenance is thus distinguished over the state of the art by employment of an effective combination of data from various sources when determining the threshold value for the at least one operating parameter being monitored. This allows for a more accurate and precise forecasting of the respective component's failure probability. In particular, maintenance reports and archives provide insights into the performance of the respective components in prior operating cycles. Moreover, the measurements from sensors arranged inside the X-ray system as well as from sensors arranged outside of the X-ray system allow for consideration of parameters crucial in determining the current operating state of the X-ray system, such as temperature, temperature gradient and air quality, including the presence and concentration of dust particles, and, most importantly, their differences/ratios from inside and outside the X-ray system. This particular combination of data leads to an increased accuracy in determining the actual state of the X-ray system as well as predicting the respective component's failure probability at any given time. Specifically, the suggested combination of considered data improves predictive maintenance of the X-ray system even when those operating conditions, which known predictive maintenance methods critically rely on, are within normal parameter ranges, still far from the pre-defined critical thresholds. The method according to the present invention considers these aspects when obtaining and/or adapting the threshold values for all the relevant operating parameters. Thus, when comparing the monitored values of a particular operating parameter with its corresponding threshold value, one obtains a sound estimation of the respective component to which said operating parameter relates.

In accordance with the definition of step ii., to each monitored operating parameter corresponds an obtained (calculated) threshold value, and to each monitored further operating parameter corresponds a predefined threshold value. According to a preferred embodiment of the method, step i. includes the gathering of data from an operating system of the X-ray system, preferably including number, type, and content of signals and messages generated by the operating system.

Such data from the X-ray system's operating system may, at first glance, seem not to be related to the X-ray system's current operating state. However, by considering data on software processes and feedback messages from the X-ray system, for instance computer log files, diagnostic files or error files generated by the X-ray system, the calculation (step ii.) and/or the dynamic adaption (step iii.) of the threshold values becomes more involved and comprehensive, as compared to the state of the art where such threshold values are either pre-set or calculated without taking into account any data from the X-ray system's operating system. In other words, the threshold adjustment and the status forecast become more accurate when the results of the operating system's load analysis, number, type and content of signals and messages are taken into account.

According to another preferred embodiment of the method, said method is performed in real time during operation of the X-ray system. In particular, steps i., ii., iii., iv., v., and vi. of said method may be performed continuously during operation of the X-ray system. However, it is also possible that only steps i., iii., iv., v., and vi. of said method may be performed continuously during operation of the X-ray system. This could be the case when the threshold value(s) is/are provided as (a) predefined value(s).

Thereby, the method enables real-time predictive maintenance of X-ray systems, allowing for continuous monitoring of the system's current operating state and of its component's failure probability.

Preferably, the method according to the present invention is performed (continuously) for at least one operating parameter and/or further operating parameter of each critical component of the X-ray system.

In this way, all critical components of the X-ray system - i.e. those components of the X-ray system which are prone to failure (e.g due to their high loads) and thus are of interest for the predictive maintenance - may be monitored with respect to their failure probability. Consequently, it becomes possible to accurately predict which part or component of the X-ray system will fail at which point in time, which allows for a smooth and uninterrupted operation of facilities relying on the operability of X-ray systems e.g. security checkpoints at airports.

An objective of the present invention is achieved by a system comprising an X-ray system, a device for retrofitting the X-ray system, and a data processing unit, said system being adapted to execute the method according to the present invention.

Said system enables predictive monitoring for already existing X-ray systems of various types and models, irrespective of whether these X-ray systems are already equipped with the sensors, data acquisition and processing units, and/or other means required for executing the method according to the present invention.

In particular, existing X-ray systems may be retrofitted with said device which preferably comprises means for gathering data relating to the operation of the X-ray system.

These means may be adapted for gathering data from maintenance reports and/or maintenance archives relating to the X-ray system and/or from sensors arranged inside the X-ray system as well as from sensors arranged outside of the X-ray system. Thus, said device provides for acquisition of all data necessary for obtaining (step ii.) and/or dynamically adapting (step iii.) the respective threshold values for the monitored operating parameters, comparison of which determines the failure probability of the respective component.

According to a preferred embodiment of said system, it comprises at least one sensor for recording at least one operating parameter of a component of the X-ray system.

Such sensors may be adapted to be arranged inside or outside of the X-ray system. Moreover, sensors which are positioned inside the X-ray system may be arranged inside or outside the respective component(s) relating to the monitored operating parameter(s). Data from these sensors may be employed in steps i., ii., iii., iv., v. and/or vi. of the method according to the present invention. By means of a system according to this preferred embodiment of the invention, even such existing X-ray systems may be provided with predictive maintenance, which do not themselves feature any internal (built-in) sensors, which can be arranged inside or outside of the X-ray system.

According to another preferred embodiment of said system, it comprises means for exchanging data with an operating system and/or with internal sensors of the X-ray system.

In this way, data from the X-ray system's operating system and/or the X-ray system's internal (built-in) sensors, which can be arranged inside or outside of the X-ray system, may be made available for employment in the method according to the present invention. In particular, data relating to software processes and feedback messages from the X-ray system, for instance computer log files, diagnostic files or error files generated by the X-ray system, may be accessed by said means for exchanging data. Additionally or alternatively, data from internal sensors of the X-ray system, measuring various operating parameters of the X-ray system, may be accessed by said means for exchanging data. Thus, the system according to this preferred embodiment of the invention facilitates improved predictive maintenance of the system's X-ray system by increasing the amount of data that can be considered in the method according to the present invention.

According to another preferred embodiment of said system, at least steps ii., iii., v., and vi. of the method according to the present invention are performed by the data processing unit. In cases where predefined threshold values for the monitored operating parameters are provided, said data processing unit is provided with these predefined threshold values.

Thus, systems according to this preferred embodiment do not require any further means for data processing. Since the gathering of required data (step i.) and the monitoring of the respective operating parameters (step iv.) may be handled by the means for exchanging data, where applicable in cooperation with the internal sensors of the X-ray system, and or by the system's at least one sensor for recording at least one (further) operating parameter of the X-ray system, the method according to the present invention may readily be performed. According to yet another preferred embodiment of said system, the data processing unit is realised as an independent network unit in communication with the device for retrofitting the X-ray system.

For instance, said data processing unit may be located at or realized by a remote data centre, which could additionally comprise servers and other IT infrastructure. In such embodiments, the failure probability may be determined off-site of the facility comprising the respective X-ray system, so that no additional processing power is required on-site.

In an alternative embodiment, the data processing unit is an integral part of the device for retrofitting the X-ray system.

As a result, the system according to the invention may operate independently and (continuous) predictive maintenance of the respective X-ray system can be ensured during the operation of said X-ray system.

BRIEF DESCRIPTION OF THE FIGURES

A brief description of the invention using figures of an exemplary embodiment now follows. Thereby shows:

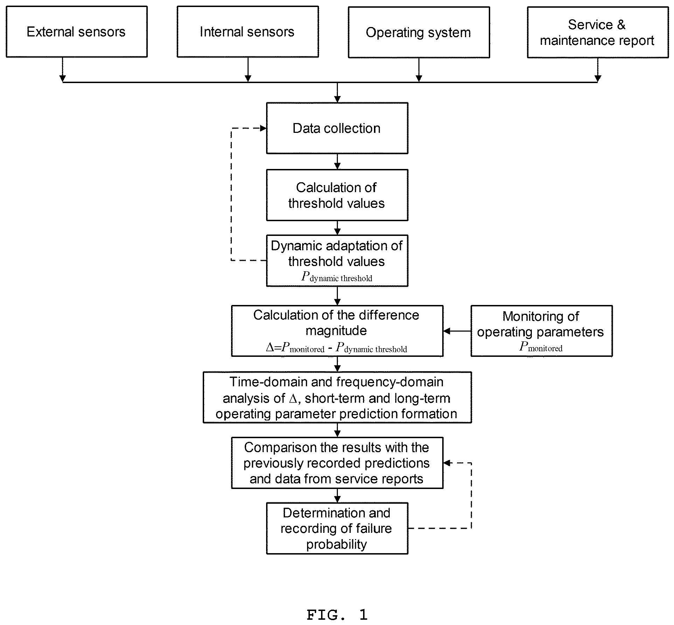

FIG. 1 a schematic overview of the method according to the present invention

WAYS OF CARRYING OUT THE INVENTION

Fig. 1 schematically illustrates an embodiment of the method according to the present invention.
An X-ray system, for which predictive maintenance is desired, comprises internal (built-in) sensors for measuring certain operating parameters of the X-ray system, whereas those internal sensors may be located inside and/or outside of the X-ray system. These operating parameters can, for instance, relate to temperature, air humidity, current, or voltage.
Moreover, additional (retrofitted) external sensors may be arranged inside or outside the X-ray system, in order to measure the same or additional operating parameters mentioned above. These additional sensors may be realised as part of a device for retrofitting the X-ray system of a system according to the invention. Said system according to the invention comprises the X-ray system, said device for retrofitting the X-ray system, and a data processing unit. Said system according to the invention may further comprise means for exchanging data with an operating system and/or with internal sensors of the X-ray system.
Furthermore, the operating system holds data on software processes and feedback messages from the X-ray system, for instance computer log files, diagnostic files or error files generated by the X-ray system. From this, one may also extract information regarding the number, type, and content of signals and messages which have been generated by the operating system during operation of the X-ray system.
In addition, service or maintenance reports hold data relating to previous maintenance works on the X-ray system. In particular, these maintenance reports may hold information on how long a service period of a particular component of the X-ray system actually was, before said component had to be replaced, and what was the reason for the failure of that particular component.
In a first step of the method according to the present invention, data from maintenance reports and/or maintenance archives relating to the X-ray system and from sensors arranged inside the X-ray system as well as from sensors arranged outside of the X-ray system is collected. According to the embodiment illustrated by Fig. 1, this step also includes data gathering from the X-ray system's operating system. This step may be performed by said device, comprising the external sensors, and/or by the means for exchanging data with an operating system and/or with internal sensors of the X-ray system.
In a second step, the collected data is statistically processed in order to obtain a threshold value for each operating parameter of the component, which is considered in the method for predictive maintenance according to the invention. In other embodiments of the method, a predefined threshold value for at least one operating parameter of the component may be provided.
In a third step, the obtained threshold values are dynamically adapted based on a difference between and/or a ratio of sensor measurements from the inside of the X-ray system and from the outside of the X-ray system. However, in other embodiments, said threshold adaption may also involve some/all of the remaining data which has been gathered in step i. This dynamic adaption happens multiple times or regularly during the operation of said X-ray system, in order to adapt the threshold values based on various changing operating conditions. For instance, if the workload of one X-ray system suddenly changes (e.g. due to an additional X-ray system commencing operation in the respective facility), this will have an impact on some components wear, which will be considered in the predictive maintenance method according to the invention by corresponding adjustments of the respective threshold values.

In a fourth step, which - generally speaking - may be performed in any order of the method according to the present invention, the operating parameters of the components of interest are monitored in order to be considered in the predictive maintenance method according to the present invention. This may be done by the system's means for exchanging data, if applicable in cooperation with the internal sensors of the X-ray system, and/or by the external sensors of the system's device.

In a fifth step, the values of the monitored operating parameters are compared to the corresponding threshold values. This comparison may be performed by a data processing unit of the system according to the present invention, either off-site or on-site of the X-ray system.

In a sixth step, the failure probabilities of the components of interest are determined, based on said comparison of the respective operating parameters and their corresponding threshold values. For instance, the failure probability may be determined as a function of the difference or ratio of all monitored operating parameters, relating to that particular component, and their corresponding threshold values. Alternatively, the failure probability may be determined as follows:
Comparing the values of the monitored operating parameters $P_{monitored}$ to their corresponding dynamic threshold values $P_{dynamic\ threshold}$ may be achieved by means of the function

$$\Delta = P_{monitored} - P_{dynamic\ threshold}.$$

Subsequently, first and second partial time derivatives $\dot{\Delta}$ and $\ddot{\Delta}$ of this difference magnitude $\Delta$ may be determined and a short-term prediction STP may be obtained by means of (numerical) extrapolation in near-real-time,

$$STP = f_t\big(\Delta, \dot{\Delta}, \ddot{\Delta}, t\big).$$

Analogously, a long-term prediction LTP may be obtained by means of (frequency-domain) Fourier transformation,

$$LTP = f_{FT}\big(\Delta, \dot{\Delta}, \ddot{\Delta}, t\big).$$

Moreover, machine learning algorithms may be employed to examine all short and long-term prediction values (STP, LTP) for all operating parameters $P_{monitored}$ for given periods of time, and to estimate a correlation factor between them. These results are subsequently compared to previously recorded predictions corresponding to the data from service or maintenance reports. Thereby, a probabilistic numerical value is obtained representing the current prediction for the current operating status (i.e. health state) of the X-ray system and/or its monitored components.

However, since operating parameters are constantly measured, since the corresponding threshold values are dynamically determined, and since the calculation of the failure probabilities may be performed in real-time, said probabilistic numerical value will change with time, thereby reflecting changes in operating conditions of the X-ray system and/or of the data considered by the prediction models.

**Claims**

1. Method for predicting a failure probability of a component of an X-ray system, comprising the following steps:

   i. gathering data from maintenance reports and/or maintenance archives relating to the X-ray system and from sensors arranged inside the X-ray system as well as from sensors arranged outside of the X-ray system;
   ii. statistically processing said data in order to obtain at least one threshold value for at least one operating parameter of the component, and/or providing at least one predefined threshold value for at least one further operating parameter of the component;
   iii. dynamically adapting the at least one threshold value based, at least, on a difference between and/or a ratio of sensor measurements from the inside of the X-ray system and from the outside of the X-ray system;
   iv. monitoring the at least one operating parameter of the component and/or the at least one further operating parameter of the component;
   v. comparing the values of the monitored at least one operating parameter and/or the monitored at least one further operating parameter to the corresponding threshold value;
   vi. based on said comparison, determining the failure probability of the component.

2. Method according to claim 1, **characterised in that** step i. includes the gathering of data from an operating system of the X-ray system, preferably including number, type, and content of signals and messages generated by the operating system.

3. Method according to claim 1 or 2, **characterised in that** said method is performed in real time during operation of the X-ray system.

4. Method according to claims 1, 2 or 3, **characterised in that** said method is performed, preferably continuously performed, for at least one operating parameter and/or further operating parameter of each critical component of the X-ray system.

5. System comprising an X-ray system, a device for retrofitting the X-ray system, and a data processing unit, said system being adapted to execute the method according to any of claims 1 to 4.

6. System according to claim 5, **characterised in that** said device comprises means for gathering data relating to the operation of the X-ray system.

7. System according to claim 5 or 6, **characterised in that** it comprises at least one sensor for recording at least one operating parameter and/or further operating parameter of a component of the X-ray system.

8. System according to any of claims 5 to 7, **characterised in that** it comprises means for exchanging data with an operating system and/or with internal sensors of the X-ray system.

9. System according to any of claims 5 to 8, **characterised in that** at least steps ii., iii., v., and vi. of the method according to claim 1 are performed by the data processing unit.

10. System according to claim 9, **characterised in that** the data processing unit is realised as an independent network unit in communication with the device for retrofitting the X-ray system.

11. System according to claim 9, **characterised in that** the data processing unit is an integral part of the device for retrofitting the X-ray system.

| External sensors | Internal sensors | Operating system | Service & maintenance report |
|---|---|---|---|

Data collection

Calculation of threshold values

Dynamic adaptation of threshold values
$P_{\text{dynamic threshold}}$

Calculation of the difference magnitude
$\Delta = P_{\text{monitored}} - P_{\text{dynamic threshold}}$

Monitoring of operating parameters
$P_{\text{monitored}}$

Time-domain and frequency-domain analysis of $\Delta$, short-term and long-term operating parameter prediction formation

Comparison the results with the previously recorded predictions and data from service reports

Determination and recording of failure probability

FIG. 1

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

Application Number

EP 20 19 0781

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/179765 A1 (KONINK PHILIPS NV) 26 September 2019 (2019-09-26) * paragraphs [0004], [0010] - [0012], [0025], [0026], [0028], [0030] - [0036]; figure 1 *<br>----- | 1-11 | INV.<br>H05G1/54<br>G16H40/40<br>A61B6/00 |

TECHNICAL FIELDS
SEARCHED (IPC)

H05G
G16H
A61B

### INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 26 April 2021 | Krauss, Jan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

&: member of the same patent family, corresponding
document

EPO FORM 1503 03.82 (P04E07)

Europäisches
Patentamt

European
Patent Office

Office européen
des brevets

**INCOMPLETE SEARCH**
**SHEET C**

Application Number

EP 20 19 0781

Claim(s) completely searchable:
-

Claim(s) searched incompletely:
1-11

Reason for the limitation of the search:

As explained in the accompanying Search Opinion and the Communication
pursuant to Rule 63(1) EPC dated 29.01.2021, objections under i.a. Arts.
84 and 84 EPC arise in view of all present claims.
Insofar, the search has been restricted to the literal wording of the
claims and only one exemplary document has been cited.

## ANNEX TO THE EUROPEAN SEARCH REPORT
## ON EUROPEAN PATENT APPLICATION NO.

EP 20 19 0781

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2021

| Patent document cited in search report | Publication date | Patent family member(s) | Publication date |
|---|---|---|---|
| WO 2019179765 A1 | 26-09-2019 | CN 111886653 A<br>EP 3769318 A1<br>US 2020365262 A1<br>US 2021012891 A1<br>WO 2019179765 A1 | 03-11-2020<br>27-01-2021<br>19-11-2020<br>14-01-2021<br>26-09-2019 |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6453009 B2